(19) Europäisches Patentamt
European Patent Office
Office européen des brevets

(11) **EP 4 592 958 A1**

(12) **EUROPEAN PATENT APPLICATION**

(43) Date of publication:
**30.07.2025 Bulletin 2025/31**

(21) Application number: **24154353.7**

(22) Date of filing: **29.01.2024**

(51) International Patent Classification (IPC):
**G06T 11/00** (2006.01)

(52) Cooperative Patent Classification (CPC):
**G06T 11/005;** G06T 2211/421; G06T 2211/424;
G06T 2211/432

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Carl Zeiss X-Ray Microscopy, Inc.
Dublin, California 94568 (US)**

(72) Inventors:
• **ANDREYEV, Andriy**
**94568 Dublin, California (US)**
• **YANG, Faguo**
**94568 Dublin, California (US)**
• **OMLOR, Lars**
**94568 Dublin, California (US)**
• **ANDREW, Matthew**
**94568 Dublin, California (US)**

(74) Representative: **HGF
HGF Europe LLP
Neumarkter Straße 18
81673 München (DE)**

(54) **ITERATIVE RECONSTRUCTION METHOD AND SYSTEM FOR INTERIOR TOMOGRAPHY**

(57) An X-ray micro tomography system provides the ability to proscriptively determine regularization parameters for iterative reconstruction of a sample, from projection data of the sample. This allows a less experienced operator to determine the regularization parameters with adequate precision.

Fig. 4

Fig. 5

**Description**

BACKGROUND OF THE INVENTION

X-ray micro tomography systems provide high-resolution, non-destructive imaging of internal structures in samples. The systems are utilized in a variety of industrial and research applications, such as mining, manufacturing, materials science, clinical research, and failure analysis, in examples. The systems provide the ability to visualize features in samples without the need to cut and slice the samples.

X-ray micro tomography systems include X-ray projection systems that produce projection data of the sample and computer systems that reconstruct tomographic images of the sample from the projection data. In operation, the X-ray projection system executes a scan of a sample at different angles to produce the projection data. During a scan, X-rays are directed to the sample, and are absorbed or scattered by the sample as the X-rays travel through the sample. The X-rays not absorbed or scattered away are transmitted through and modulated by the sample. A detector system receives the transmitted X-rays, and creates an image representation, in pixels, of the received X-rays. The series of X-ray projections at different angles produced from the scan form the projection data of the sample. Then, the computer system accesses the projection data, and applies image reconstruction algorithms to the projection data to reconstruct volume datasets of the sample. A volume dataset is a three dimensional (3D) representation of the entire sample, and a slice is a two dimensional (2D) cross-sectional image of the sample based on the volume dataset.

The most commonly used reconstruction algorithms fall into a class of reconstruction techniques termed analytical reconstruction. The objective is to find a closed-form solution to the problem of reconstructing an object's internal structure from its projections. The most common analytical method is filtered back projection (FBP). The projections are first processed using a high-frequency amplification filter, usually a ramp filter, in the frequency domain. Then, each filtered projection is "smeared" back onto the imaging plane as if each data point emits back uniformly in the shape of the original beam. These back projections from all angles are summed up to produce the reconstructed image, which approximates the object's internal structure. The filtering and back projection operations collectively help in obtaining a more accurate and less blurred reconstruction of the original object.

Iterative reconstruction is another approach that reconstructs slices from successive estimates of the projection data forming each slice. Multiple iterations of the estimated projection data for each slice are executed for this purpose. During each iteration, the estimated projection data are compared to the actual (e.g. measured) projection data. The result of each comparison is used to make corrections to the current image volume estimate, thereby creating a new estimate of the projection data. When the estimated projection data no longer require corrections, a final volume dataset is reconstructed from the estimated projection data. Exemplary iterative reconstruction algorithms include algebraic reconstruction technique (ART), simultaneous reconstruction technique (SIRT) and iterative least-squares technique (ILST). The algorithms typically differ in the way the measured and estimated projection data are compared and the kind of correction applied to the current estimate.

Analytical reconstruction and iterative reconstruction modalities have advantages and disadvantages. FBP, for example, provides good image quality with relatively low processing overhead, which increases throughput and reduces cost. A disadvantage of FBP is susceptibility to image noise in the projection data. Advantages of iterative reconstruction include generally improved image quality, less susceptibility to image noise, reduced image artifacts, and the ability to reconstruct an optimal image in the case of incomplete projection data. Disadvantages of iterative reconstruction include complexity associated with selection of regularization parameters applied to the iterative reconstruction algorithm, longer processing time, and increased computational cost.

Nevertheless, one common problem is that iterative reconstruction algorithms require complete data and do not work well with interior tomographies. Interior tomography happens when the scanner acquisition geometry is set up in such a way that the whole object is not fully represented in all or some projection views. If left uncorrected, it typically results in non-uniformities, distortions and shading artifacts, undermining benefits from the iterative reconstruction. And, interior tomographies are especially common when using X-ray micro tomography systems due to the constant need to increase the spatial resolution through high magnification and limited FOV. Since iterative image reconstruction algorithms consist of repeated sequences of forward and back projections, they are much more susceptible to the missing data in the interior tomography problem.

Due to these challenges, specialized iterative reconstruction algorithms, often incorporating a priori information or additional constraints, are developed for interior tomography. Some approaches combine both iterative and analytical methods to offer a trade-off between computational efficiency and image quality.

An example is described in Arkadu et al., "Fast iterative reconstruction of data in full interior tomography," Journal of Synchrotron Radiation, Jan 1;24(Pt 1):205-219, 2017. They tried to solve the problem by using analytical reconstruction such as FDK to reconstruct an initial image estimate within the circular field of view, then perform forward projection to generate a new set of data minimally affected by interior tomography problem. The iterative reconstruction algorithm would then be applied to forward projected data directly, reconstructing image volume corrected for interior tomography.

There are also other known methods to solve the interior tomography problem. U.S. Patent No. 9,237,874, by B. De Man et al., concerns employs a hybrid detector that includes both high-resolution and low resolution portions. Region of interest (ROI) imaging with the high resolution portion of the detector can be used to correct for interior tomography by using full scan overview from entire detector. U.S. Patent No. 8,811,700, by G. Wang et al. requires a priori knowledge about the content of internal ROI in order to provide assumptions for compressive sensing model. U.S. Patent No. 11,457,878 by H. Kudo et al. describes a method for generating corrected interior tomography reconstruction by requiring two data acquisitions, one consisting of line integrals passing through the interior ROI, and at least a segment of line integrals passing through entire object. U.S. Patent Appl. No. US 2016/0282432 by G. Wang et al. requires data acquisitions from several imaging modalities (not just CT, but ultrasound, SPECT, MR, etc.).

## SUMMARY OF THE INVENTION

The present invention concerns iterative image reconstruction of interior tomographies. This extends the improved performance of iterative reconstruction into a domain in which it often exhibits problems such as non-uniformities, distortions and shading artifacts. At the same time, assumptions as with other techniques are not required. Only a single set of data is need to derive the suitable correction.

In general according to one aspect, the invention features an X-ray micro tomography system. The system includes an X-ray microscopy system including an X-ray source system for generating an X-ray beam and a detector system for detecting the X-ray beam after transmission through a sample to generate projection data for interior tomographies, where the whole sample is not fully visible at every projection view. A computer system executes analytical reconstruction to create an analytical volume data set using the projection data and using the analytical volume data set to generate analytical forward projection data and to correct the analytical forward projection data and executing iterative reconstruction using the corrected analytical forward projection data.

Preferably, the analytical reconstruction implements filtered backprojection. FDK is preferred. In addition, a region of interest of the sample is ideally extended in Z direction.

In the current embodiment, the analytically reconstructed volume is clipped based on a field of view.

The forward-projected data and the original projections can be filtered such as with a box filter to reduce noise.

In a current example, the analytical projections and the original projections are filtered and their ratio is used to scale the projection data to produce the corrected projection data. Specifically, their difference is subtracted from the projection data to produce the corrected projection data.

In general, according to one aspect, the invention features a method for an X-ray micro tomography system. As is common, an X-ray beam is generated and detected after transmission through at least a part of a sample to generate projection data. A computer system executes analytical reconstruction to generate an analytical volume data set using the projection data and uses the analytical volume dataset to generate analytical forward projection data. The analytical forward projections and the projection data are combined to generate corrected analytical projection data. Then, an interior tomography corrected volume dataset of at least the part of the sample is generated by executing iterative reconstruction using the corrected analytical forward projection data.

In general, according to one aspect, the invention features an X-ray micro tomography system comprising an X-ray microscopy system and a computer system. As is typical, the X-ray microscopy system will have an X-ray source system for generating an X-ray beam and a detector system for detecting the X-ray beam after transmission through a sample to acquire standard projection data which may be an internal tomography. The computer then executes analytical reconstruction and uses the analytical reconstruction to correct the projection data for the internal tomographies. Then iterative reconstruction is performed on the corrected projection data.

In embodiments, the analytical reconstruction implements filtered back projection, such as FDK reconstruction, with an extended reconstructed volume in Z direction.

In embodiments, the analytical reconstruction implements filtered back projection, such as FDK reconstruction, with image volume with uniformity correction applied.

Then, preferably the analytical volume dataset from the analytical reconstruction is clipped based on a field of view and is forward projected to create analytical projections.

These analytical projections can be filtered such as with a filter, such as a box filter, to reduce noise.

In one embodiment, box filtered analytical projections and the original projections are used to scale the original projection data to produce the corrected projection data.

In one embodiment, filtered analytical projections and the original projections are subtracted from the original projection data to produce the corrected projection data. At this point, the corrected data can be reconstructed with iterative image reconstruction algorithm to create iteratively reconstructed image which will be no longer be affected by interior tomography artifacts.

In general, according to another aspect, the invention features a method for an X-ray micro tomography system comprising generating an X-ray beam, detecting the X-ray beam after transmission through the sample to generate projection data, and then executing analytical reconstruction and using the analytical reconstruction to correct the projection data for the internal tomographies and then executing iterative reconstruction using the corrected projection data.

The above and other features of the invention including various novel details of construction and combinations of parts, and other advantages, will now be more particularly described with reference to the accompanying drawings and pointed out in the claims. It will be understood that the particular method and device embodying the invention are shown by way of illustration and not as a limitation of the invention. The principles and features of this invention may be employed in various and numerous embodiments without departing from the scope of the invention.

BRIEF DESCRIPTION OF THE DRAWINGS

In the accompanying drawings, reference characters refer to the same parts throughout the different views. The drawings are not necessarily to scale; emphasis has instead been placed upon illustrating the principles of the invention. Of the drawings:

Figs. 1A and 1B are schematic top views of an x-ray micro tomography system showing a full tomography setup (Fig. 1A) and an interior tomography setup (Fig. 1B);

Figs. 2A and 2B are slices taken from volume datasets calculated using analytical reconstruction (Figs. 2A) and iterative reconstruction (Figs. 2B) of an interior tomography setup.

Fig. 3 is a schematic diagram of an X-ray micro tomography system to which the methods of the present invention are applicable;

Fig. 4 is a flow diagram showing a hybrid analytical and iterative reconstruction method according to the present invention;

Fig. 5 is a schematic side view showing how FDK projections are extended in the Z direction of the sample for maximum cone angle intersection with cylindrical ROI; and

Figs. 6A and 6B are slices taken from volume datasets calculated using iterative algorithm with no interior tomography correction (Figs. 6A) and iterative algorithm with interior tomography correction (Figs. 6B).

DETAILED DESCRIPTION OF THE PREFERRED EMBODIMENTS

The invention now will be described more fully hereinafter with reference to the accompanying drawings, in which illustrative embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including component or subsystem, is referred to and/or shown as being connected or coupled to another element, it can be directly connected or coupled to the other element or intervening elements may be present.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

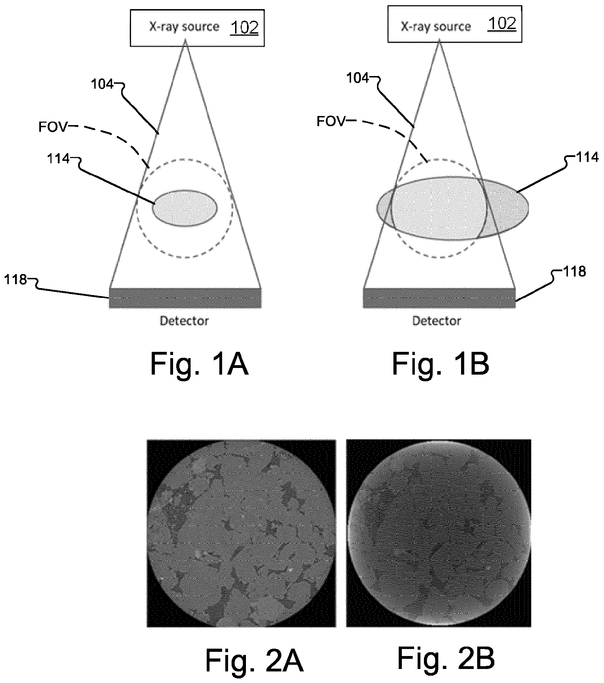

Figs. 1A and 1B show an X-ray tomography setup illustrating the difference between a full tomography and an interior tomography.

In the basic configuration, the X-ray source 102 emits a cone shaped beam 104 that is common in X-ray micro tomography systems especially when employing laboratory sources. As shown in Fig. 1A, the beam 104 compasses the entire field of view (FOV) and specifically an entire cross section of the sample 114; and the detector system 118 has a large enough extent to image the entire cross section of the sample. Fig. 1B illustrates the problem of interior topographies. The beam 104 and the extent of the detector system 118 is insufficient to capture an entire cross section of the sample 114 with the sample extending beyond the field of view.

Fig. 2A is a slice taken from a volume dataset calculated using a type of analytical reconstruction termed FDK (Feldkamp, Davis and. Kress) reconstruction and Fig. 2B is a slice taken from a volume dataset calculated using iterative reconstruction. In the case of an interior tomography, the FDK reconstructed images of interior tomographies do not suffer from the kind of problems as iterative reconstructions. Iterative image reconstruction algorithms consist of repeated sequences of forward and back projections. Therefore, they are much more susceptible to the missing data in the interior tomography problem.

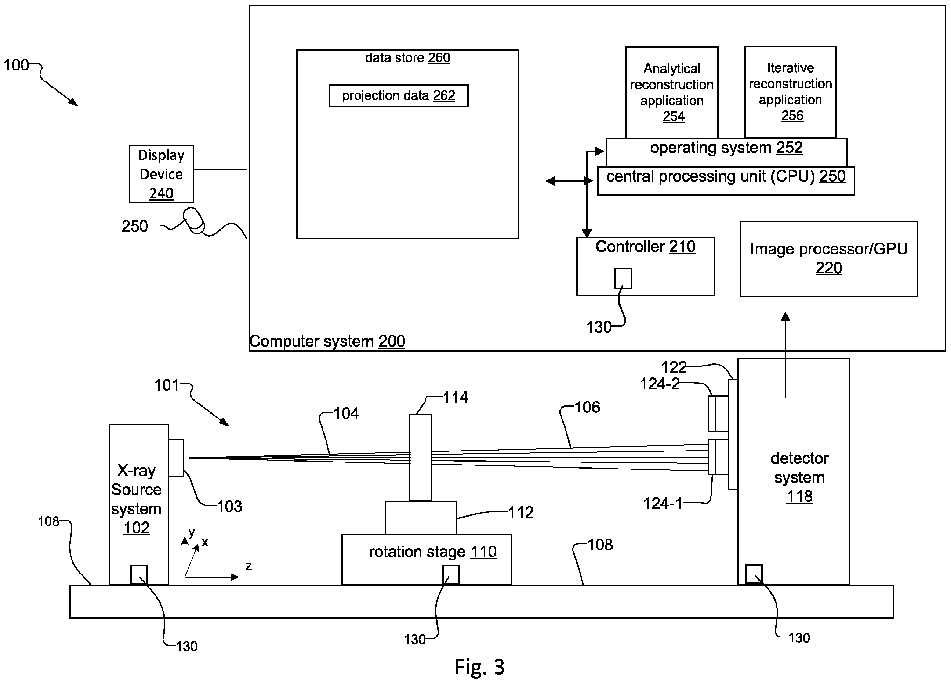

Fig. 3 is a schematic diagram of an X-ray micro tomography system 100 to which the methods and workflows of the present invention are applicable.

In general, the X-ray micro tomography system 100 combines an X-ray microscopy system 101 and a computer system 200 for receiving projections and calculating volume datasets from those projections.

The X-ray microscopy system includes the X-ray source system 102 that generates a typically polychromatic X-ray beam 104 and a rotation stage 110 with sample holder 112 for holding and rotating the sample 114 in the X-ray beam 104 from the X-ray source system 102. X-ray projections are captured by the detector system 118. The X-ray source system 102, the rotation stage 110, and the detector system 118 are mounted to a base 108 of the x-ray CT system 100. A computer system 200 typically receives and processes these projections and provides general control of the system 100. The computer system 200 or other computer will typically perform tomographic reconstruction using the X-ray projections to create volume datasets.

The X-ray source 102, in one example, is a cone-beam, polychromatic X-ray source. The polychromatic X-ray source is preferably a laboratory X-ray source because of its ubiquity and relatively low cost. Nonetheless, synchrotron sources or accelerator-based sources are other alternatives.

Common laboratory X-ray sources include an X-ray tube, in which electrons are accelerated in a vacuum by an electric field and shot into a target piece of metal, with X-rays being emitted as the electrons decelerate in the metal.

In one example, the X-ray source 102 is micro focused source, with a Tungsten target. Targets that include Molybdenum, Gold, Platinum, Silver or Copper also can be employed. Preferably, a transmissive-type target configuration of the X-ray source 102 is used in which the electron beam strikes the thin target 103 from its backside. The X-rays emitted from the other side of the target 103 are then used as the beam 104.

When the sample 114 is exposed to the X-ray beam 104, the X-ray photons transmitted through the sample form an attenuated X-ray beam 106 that is received by the detector system 118. In some other examples, an objective lens such as a zone plate lens is used to form an image onto the detector system 118 of the X-ray imaging system 100.

In the most common configuration of the detector system 118, a magnified projection image of the sample 114 is formed on the detector system 118 with a geometrical magnification that is equal to the inverse ratio of the source-to-sample distance and the source-to-detector distance. Generally, the geometrical magnification provided by the X-ray imaging system is between 2x and 100x, or more. In this case, the resolution of the X-ray image is limited by the focus spot size or virtual size of the X-ray source system 102.

To achieve high resolution, an embodiment of the X-ray micro tomography system 100 further utilizes a very high resolution detector 124-1 of the detector system 118 in conjunction with positioning the sample 114 close to the X-ray source system 102. In one implementation of the high-resolution detector 124-1, a scintillator is used in conjunction with a microscope objective to provide additional magnification in a range between 2x and 100x, or more.

Other possible detectors can be included as part of the detector system 118 in the illustrated X-ray CT system 100. For example, the detector system 118 can include a lower resolution detector 124-2. This could be a flat panel detector or a detector with a lower magnification microscope objective, in examples. Configurations of one, two, or even more detectors 124 of the detector system 118 are possible.

Preferably, two or more detectors 124-1, 124-2 are mounted on a rotating turret 122 of the detector system 118, so that they can be alternately rotated into the path of the attenuated beam 106 from the sample 114.

Typically, based on operator defined parameters, the controller 210 of the computer system 200 instructs the rotation stage 110 via the control interface 130 to move the sample 114 out of the beam path during X-ray source system 102 calibration. After completion of the calibration portion, the controller 210 moves the sample 114 back into the beam path and rotates the sample 114 relative to the beam 104 to perform the CT scan of the sample 114 and saving the projection data 262 to a datastore 260.

In one example, the computer system 200 includes an image or other acceleration processor 220 that analyzes the X-ray projections and possibly performs the calculations necessary for tomographic reconstructions created from the X-ray projections. A display device 240, connected to the computer system 200, displays information from the X-ray CT system 100. An input device 250 such as a touch screen, keyboard, and/or computer mouse enables interaction between the operator, the computer system 200, and the display device 240.

Using user interface applications executing on the computer system 200 that display their interfaces on the display device 240, in one example, the operator defines/selects CT scan, acquisition and/or calibration parameters.

These include X-ray acceleration voltage settings, and settings for defining the X-ray energy spectrum of the scan and exposure time on the X-ray source system 102. The operator also typically selects other settings such as the number of X-ray projection images to create for the sample 114, the angles to rotate the rotation stage 110 for rotating the sample 114 for an X-ray CT scan in the x-ray beam 104 and the distances between the source 102, rotation stage 110, and the detector 118.

The computer system 200, with the assistance of its image processor 220, accepts the image or projection information from the detector system 118 associated with each rotation angle of the sample 114. Often the image processor 220 combines the projection images using reconstruction algorithms to create 3D tomographic reconstructed volume information for the sample.

According to the invention, the computer system 200 executes both an analytical reconstruction application 254 along with an iterative reconstruction application 256 for operating on the projection data 262.

The reconstruction applications 254, 256 runs on top of the operating system 252. The operating system 252, in turn, is executed by the computer's central processing unit(s) (CPU) 250.

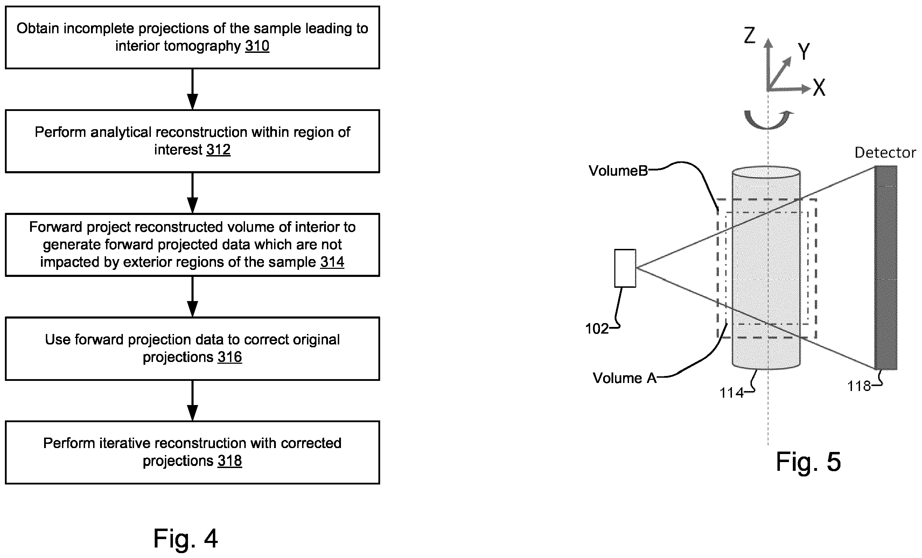

Fig. 4 shows the inventive method. It is based on the assumption that analytical single back projection methods such as FDK are less sensitive to the interior tomography problem due to localized nature of the ramp filter. Therefore, the FDK reconstructed images are employed as prior knowledge and used to correct the raw projection data facilitate the convergence of iterative reconstruction.

In more detail, in step 310, a series of projections 262 of the sample 114 are captured using the X-ray micro tomography system 100 and stored to the data store 260. These projections are or include interior projections and do not cover the whole sample or object, meaning not every subregion of the object is imaged at every projection angle, as shown in Fig. 1B..

In step 312, analytical reconstruction is performed within the field of view covering a portion of a cross-section of the sample 114 by employing the analytical reconstruction application 254. Per Fig. 5, the reconstruction volume is extended in Z-direction as shown (volume B) to ensure maximum possible coverage which will be important when the volume will be forward projected in the next step. It is also important to correct for the sensitivity fall off in peripheral regions. For reconstruction it is also recommended to extrapolate the projection values in the edges to further minimize truncation errors. Extrapolation can be done by either repeating the last measured pixel several times, or by other similar methods (repeating the mean value of the group of edge pixels, or by using dampening function). With the X-ray micro tomography system 100 illustrated, the FDK reconstruction is preferably utilized due to the cone beam geometry.

In step 314, the FDK-reconstructed image volume dataset is clipped to an interior tomography field of view (in a circular fashion) and forward projected matching the acquisition geometry that was used to acquire original data. This yields forward projected projection data that is virtually unimpacted by the exterior regions of the object. This is shown in Fig. 5. The typical volume is defined by central rotation axis with the X-ray cone beam (VolumeA). Here, it is extended in the Z direction until the edge of the FOV intersects with cone beam (VolumeB) as is preferred in the inventive method. The FDK projections are extended in the Z direction of the sample 114 for maximum cone angle intersection with cylindrical FOV and corrected for non-uniform sensitivity arising from cone-beam geometry of the system 100.

In step 316, the original projections are corrected to create interior tomography corrected projections based on the estimate of the sample from the volume dataset from the FDK analytical reconstruction.

Interior tomography corrected projection data $p'_j$ are derived from the original projections $p_j$ on a pixel-per-pixel basis as:

$$p'_j = p_j \cdot \max\left(0, \min\left(\frac{\min\left(\overline{p_{j,FDK}}, \overline{p_j}\right)}{\overline{p_j}}, 1\right)\right) \tag{1}$$

where $p_{j,FDK}$ are projections with pixel (projection bin) index j, created from the forward projected FDK projections which are clipped to the circular ROI. operator indicates NxN box filter operation centered around pixel j to minimize random noise fluctuations effect, the value of N in pixels can be found empirically, for example, a value of N=3 can be a suitable choice.

The formula also indicates that the correction factor must be between 0 and 1. In general, the min and max operations denote that correction factor should not be negative and should not exceed the value of 1 (as there is no physical meaning for that).

An alternative form of subtraction-based formula is used in other embodiments such as:

$$p'_j = \max\left(p_j - \max\left(\overline{p_j} - \overline{p_{j,FDK}}, 0\right), 0\right) \tag{2}$$

where all notations are the same as in (1).

Finally, in step 318, iterative reconstruction is performed using the iterative reconstruction application 256. The iterative reconstruction operates with using the interior tomography corrected projection data p' according to either equation (1) or (2).

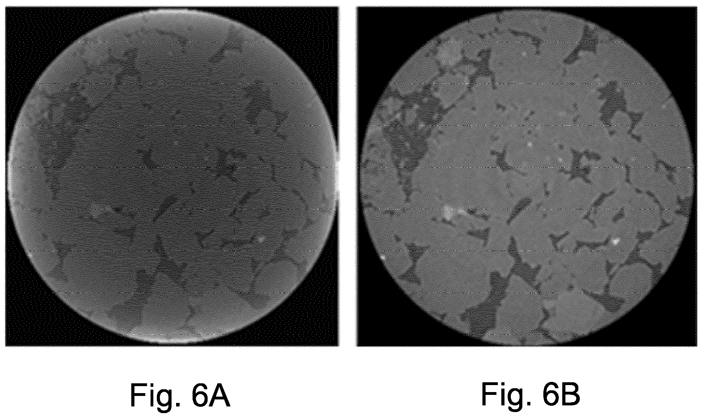

Figs. 6A is an image reconstructed with an iterative algorithm with no interior tomography correction, and Fig. 6B an image reconstructed with an iterative algorithm with interior tomography according to equation (1). The non-uniformity and distortion caused by data incompleteness are effectively eliminated by the proposed method.

While this invention has been particularly shown and described with references to preferred embodiments thereof, it will be understood by those skilled in the art that various changes in form and details may be made therein without departing from the scope of the invention encompassed by the appended claims.

**Claims**

1.  An X-ray micro tomography system, comprising:

    an X-ray microscopy system including an X-ray source system for generating an X-ray beam and a detector system for detecting the X-ray beam after transmission through a sample to generate projection data for interior tomographies, where the whole sample is not fully visible at every projection view; and
    a computer system for executing analytical reconstruction to create an analytical volume data set using the projection data and using the analytical volume data set to generate analytical forward projection data and to correct the analytical forward projection data and executing iterative reconstruction using the corrected analytical forward projection data.

2.  The system of claim 1, wherein the analytical reconstruction implements filtered backproj ection.

3.  The system of either of claims 1 or 2, wherein the analytical reconstruction includes FDK reconstruction.

4.  The system of any of claims 1-3, wherein a region of interest of the sample is extended in Z direction.

5.  The system of any of claims 1-4, wherein the analytically reconstructed volume is clipped based on a field of view.

6.  The system of any of claims 1-5, wherein the forward-projected data and the original projections are filtered with a box filter to reduce noise.

7.  The system of any of claims 1-6, wherein the analytical projections and the original projections are filtered and their ratio is used to scale the projection data to produce the corrected projection data.

8.  The system of any of claims 1-6, wherein the analytical projections and the original projections are filtered and their difference is then subtracted from the projection data to produce the corrected projection data.

9.  A method for an X-ray micro tomography system, comprising:

    generating an X-ray beam;
    detecting the X-ray beam after transmission through at least a part of a sample to generate projection data; and
    a computer system executing

    • analytical reconstruction to generate an analytical volume data set using the projection data,
    • using the analytical volume dataset to generate analytical forward projection data,
    • using the analytical forward projections and the projection data to generate corrected analytical projection data,
    • generate an interior tomography corrected volume dataset of at least the part of the sample by executing iterative reconstruction using the corrected analytical forward projection data.

10. The method of claim 9, wherein the analytical reconstruction implements filtered backprojection.

11. The method of either of claims 9 or 10, wherein the analytical reconstruction includes FDK reconstruction extended in Z direction.

12. The method of any of claims 9-11, wherein the analytical volume dataset is clipped based on a field of view.

13. The method of any of claims 9-12, wherein the forward-projected and the original projections are filtered with a box filter to reduce noise.

14. The method of any of claims 9-13, wherein the analytical projections and the original projections are filtered and then used to scale the projection data to produce the corrected projection data.

15. The method of any of claims 9-14, wherein the step of using the analytical forward projections and the projection data to generate corrected analytical projection data includes filtering of the analytical projection data and the original projection data and then subtracting the result from the original projection data to produce the corrected projection data.

## Fig. 1A

## Fig. 1B

## Fig. 2A

## Fig. 2B

EP 4 592 958 A1

Fig. 3

Obtain incomplete projections of the sample leading to interior tomography 310

↓

Perform analytical reconstruction within region of interest 312

↓

Forward project reconstructed volume of interior to generate forward projected data which are not impacted by exterior regions of the sample 314

↓

Use forward projection data to correct original projections 316

↓

Perform iterative reconstruction with corrected projections 318

Fig. 4

Z

Y

X

VolumeB

Detector

102

Volume A

114

118

Fig. 5

EP 4 592 958 A1

Fig. 6B

Fig. 6A

## EUROPEAN SEARCH REPORT

**Application Number**

EP 24 15 4353

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 811 700 B2 (WANG GE [US]; YU HENGYONG [US]; VIRGINIA TECH INTELL PROP [US]) 19 August 2014 (2014-08-19) | 1-6,9-13 | INV. G06T11/00 |
| A | * column 11, line 11 - line 45 * | 7,8,14, 15 | |
| | ----- | | |
| A | KRITI SEN SHARMA ET AL: "Scout-view assisted interior micro-CT", PHYSICS IN MEDICINE AND BIOLOGY, INSTITUTE OF PHYSICS PUBLISHING, BRISTOL GB, vol. 58, no. 12, 4 June 2013 (2013-06-04), pages 4297-4314, XP020246376, ISSN: 0031-9155, DOI: 10.1088/0031-9155/58/12/4297 * the whole document * | 1-15 | |
| | ----- | | |

**TECHNICAL FIELDS SEARCHED (IPC)**

G06T

The present search report has been drawn up for all claims

| Place of search | Date of completion of the search | Examiner |
|---|---|---|
| Munich | 6 July 2024 | Werling, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**EP 4 592 958 A1**

## ANNEX TO THE EUROPEAN SEARCH REPORT
## ON EUROPEAN PATENT APPLICATION NO.

EP 24 15 4353

This annex lists the patent family members relating to the patent documents cited in the above-mentioned European search report.
The members are as contained in the European Patent Office EDP file on
The European Patent Office is in no way liable for these particulars which are merely given for the purpose of information.

06-07-2024

| Patent document cited in search report | Publication date | Patent family member(s) | Publication date |
|---|---|---|---|
| US 8811700 B2 | 19-08-2014 | US 2012063659 A1 | 15-03-2012 |
| | | WO 2010121043 A2 | 21-10-2010 |

EPO FORM P0459

For more details about this annex : see Official Journal of the European Patent Office, No. 12/82

14

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9237874 B, B. De Man
- US 8811700 B, G. Wang
- US 11457878 B, H. Kudo
- US 20160282432 A, G. Wang

**Non-patent literature cited in the description**

- **ARKADU et al.** Fast iterative reconstruction of data in full interior tomography. *Journal of Synchrotron Radiation*, 01 January 2017, vol. 24 (1), 205-219